# EUROPÄISCHE PATENTSCHRIFT

(11) **EP 1 517 880 B1**
(45) Veröffentlichungstag und Bekanntmachung des Hinweises auf die Patenterteilung: **05.01.2011**
(21) Anmeldenummer: 03760673.8
(22) Anmeldetag: 20.06.2003
(51) Int. Cl.: C07C 231/02, C07C 233/09, A23L 1/226, A61K 8/42, A61Q 11/00

(54) **HERSTELLUNG VON CIS-PELLITORIN UND VERWENDUNG ALS AROMASTOFF**
PRODUCTION OF CIS-PELLITORIN AND USE AS A FLAVOURING
PREPARATION DE CIS-PELLITORINE ET SON UTILISATION COMME AROME

(30) Priorität: 20.06.2002 DE 10227462
(43) Veröffentlichungstag der Anmeldung: 30.03.2005
(73) Patentinhaber: Symrise AG, 37603 Holzminden (DE)
(72) Erfinder: GATFIELD, Ian-Lucas, 37671 Höxter (DE); LEY, Jakob, Peter, 37603 Holzminden (DE); FOERSTNER, Jan, 37603 Holzminden (DE); KRAMMER, Gerhard, 37603 Holzminden (DE); MACHINEK, Arnold, 37603 Holzminden (DE)
(74) Vertreter: Eisenführ, Speiser & Partner
(86) Internationale Anmeldenummer: PCT/EP2003/006545
(87) Internationale Veröffentlichungsnummer: WO 2004/000787

(56) Entgegenhaltungen:
- WO-A1-97/04666
- TSUKAHARA, YUHKO ET AL: 'A convenient method for the preparation of conjugated olefins from allyli acetates and aldehydes. Synthesis of pellitorine' BULLETIN OF THE CHEMICAL SOCIETY OF JAPAN Bd. 57, Nr. 10, 1984, Seiten 3013 - 3014, XP002257618
- GEDEY S. ET AL: 'Sequential resolution of ethyl 3-aminobutyrate with carboxylic acid esters by Candida antarctica lipase B' TETRAHEDRON: ASYMMETRY, ELSEVIER SCIENCE PUBLISHERS, AMSTERDAM, NL Bd. 10, Nr. 13, 02 Juli 1999, Seiten 2573 - 2581, XP004174136 ISSN: 0957-4166
- SAADALI, BOUCHRA ET AL: 'Alkamides from Artemisia dracunculus' PHYTOCHEMISTRY Bd. 58, Nr. 7, 2001, Seiten 1083 - 1086, XP002257619
- DATABASE FSTA [Online] INTERNATIONAL FOOD INFORMATION SERVICE (IFIS), FRANFURT/MAIN, DE HORSKY V.: 'Beverage based on beer; method of manufacture.', XP002277551 Database accession no. 97-1-09-h0235 -& CZ 280 972 B6 (HORSKY V.) 15 Mai 1996
- KOLLMANNSBERGER H. ET AL: 'SAEUREAMIDE IN HOCHDRUCKEXTRAKTEN AUS MUNTOKPFEFFER AMIDES FROM SUPERCRITICAL FLUID EXTRACTS OF MUNTOK-PEPPER' CHEMIE MIKROBIOLOGIE, TECHNOLOGIE DER LEBENSMITTEL, XX, XX Bd. 14, Nr. 3/4, 1992, Seiten 87 - 94, XP009026274 ISSN: 0366-7154

## Beschreibung

Die Erfindung beschreibt ein Verfahren zur Herstellung von 2*E*,4*Z*-Decadiensäure-*N-*isobutylamid (cis-Pellitorin) und dessen Verwendung als Scharfstoff und Aromastoff mit einem wärmeerzeugenden Effekt, bevorzugt in der Ernährung, der Mundhygiene oder dem Genuss dienenden Zubereitungen. Ferner betrifft die Erfindung der Ernährung, der Mundhygiene oder dem Genuss dienende Zubereitungen enthaltend 2*E*,4*Z-*Decadiensäure-*N*-isobutylamid.

Capsaicin [*N*-(4-Hydroxy-3-methoxybenzyl)-8-methyl-(6*E*)-nonensäureamid, vgl. Struktur 1, Abbildung 1] und andere Capsaicinoide sind als scharf schmeckende und wärmeerzeugende Aromastoffe aus verschiedenen Capsicum-Arten, insbesondere Chili, schon seit 1871 bekannt. Unter wärmeerzeugenden Stoffen bzw. Stoffen mit einem wärmeerzeugenden Effekt werden solche verstanden, die sensorisch einen Wärmeeindruck hervorrufen. Bei entsprechend geringer Dosierung der Capsaicinoide (der Schwellenwert liegt bei einer Verdünnung von ca. 1:10⁵) wird nur eine angenehme, neutrale Schärfe und ein Wärmegefühl im Mund wahrgenommen. Problematisch ist bei Capsaicin die hohe akute Toxizität (LD₅₀ (Maus oral) 47 mg), die die Anwendbarkeit bei der Zubereitung erschwert, sowie die bei häufiger Anwendung und Überdosierung auftretende chron. Gastritis, Nieren- und Leberschädigung (Römpp Lexikon Naturstoffchemie, Thieme 1997, S. 109). Somit besteht trotz der guten sensorischen Eigenschaften ein Bedarf an weniger problematischen Scharfstoffen. Das im weißen Pfeffer vorkommende Piperin (1-Piperoylpiperidin, vgl. Struktur 2, Abbildung 1) verursacht zwar auch einen scharfen Eindruck (Römpp Lexikon Naturstoffchemie, Thieme 1997, S. 500), zeigt aber im Vergleich zu Capsaicin eine relative Schärfe von nur ca. 1 %. Darüber hinaus besitzt Piperin einen intensiven Eigengeschmack, der an Pfeffer erinnert, so dass die Anwendung in vielen Zubereitungen nur beschränkt erfolgen kann. Kollmannsberger und Nitz (Chem. Mikrobiol. Technol. Lebensm. 1992,87-94) zeigen Ergebnisse einer Charakterisierung von Hochdruckextrakten aus Muntok-Pfeffer.

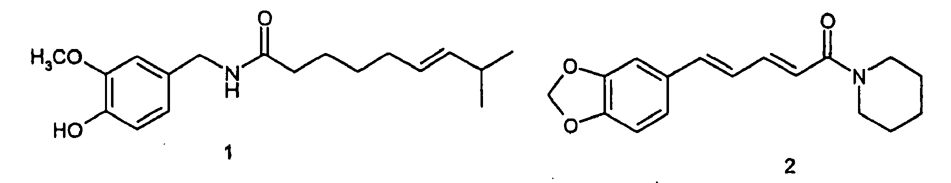

### Abbildung 1

Aufgabe der vorliegenden Erfindung war es, in der Natur vorkommende Stoffe mit einem scharfen und/oder wärmeerzeugenden Effekt sowie einem ansonsten neutralen Aromaprofil zu identifizieren, die als Aromastoffe in der Ernährung oder dem genuß dienenden Zubereitungen verwendet werden können. Weiterhin sollte ein Verfahren zur einfachen, kostengünstigen und weitgehend natürlichen Synthese entwickelt werden.

Die Erfindung betrifft daher die Herstellung von 2*E*,4*Z*-Decadiensäure-*N*-isobutylamid (cis-Pellitorin), **dadurch gekennzeichnet, dass** man einen 2E,4Z-Decadiensäureester mit Isobutylamin in Gegenwart eines Katalysators, nämlich einem Enzym mit Lipaseaktivität, wobei das Enzym als freies Protein oder auch an einen Träger assoziiert vorliegen kann, umsetzt, das Gemisch mit nicht umgesetztem 2*E*,4*Z*-Decadiensäureester gegebenenfalls einer Verseifung, bevorzugt mit einem Enzym in einem wässrigen Medium oder einer mit Wasser verdünnten Base, insbesondere bevorzugt der wässrigen Lösung von anorganischen basischen Salzen, unterzieht, optional die gebildete 2*E*,4*Z*-Decadiensäure bevorzugt extraktiv abtrennt, und anschließend das Gemisch mit physikalisch chemischen Methoden, bevorzugt durch Kristallisation, Chromatographie, Destillation oder Codestillation aufreinigt.

Die Methode wird anhand des folgenden Formelschemas verdeutlicht:

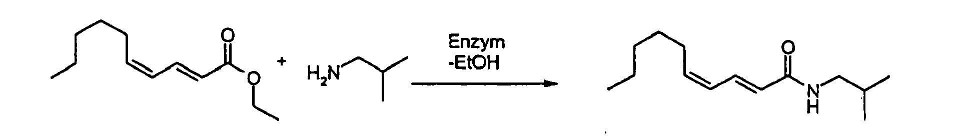

Überraschenderweise wurde festgestellt, dass das erfindungsgemäße Verfahren einen sehr einfachen Zugang zu großen, leicht zu reinigenden Mengen des gewünschten 2*E*,4*Z*-Decadiensäure-*N*-isobutylamids ermöglicht. Die bisher in der Literatur beschriebenen Synthese ist dagegen vielstufig; in schlechter Ausbeute konnte cis-Pellitorin neben trans-Pellitorin dünnschichtchromatografisch isoliert werden; bei der beschriebenen Synthese wurde u.a. das toxisch Selendioxid als Reagens verwendet (Bull. Chem. Soc. Jpn., Jahrg. 1984, Bd. 57, Seiten 3013ff.).

2*E*,4*Z*-Decadiensäure-*N*-isobutylamid im Sinne der Erfindung ist reines 2*E*,4*Z-*Decadiensäure-*N*-isobutylamid oder eine Mischung von mindestens 80 Gew.-% 2*E*,4*Z*-Decadiensäure-*N*-isobutylamid und mindestens zwei weiteren *N*-Isobutylamiden der Decansäure, 2*E*-Decensäure, 2*E*,4*E*-Decadiensäure, 2*Z*,4*E*-Decadiensäure, 2*Z*,4*Z*-Decadiensäure, 2*E*,4*Z*,7*Z*-Decatriensäure, 3*Z*,5*E*-Decadiensäure oder 3*Z*,S*E*,7*Z*-Decatriensäure.

2E,4Z-Decadiensäureester im Sinne der Erfindung sind Ester der 2E,4Z-Decadiensäure und
aliphatischer einwertiger Alkohole mit 1 bis 20 C-Atomen, insbesondere aber Methanol, Ethanol, 1-Propanol, 2-Propanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanoyl, 3-Hexanol, 1-Heptanol, 2-Heptanol, 3-Heptanol, 4-Heptanol, 1-Octanol, 2-Octanol, 1-Nonanol, 1-Decanol, 1-Undecanol, 1-Dodecanol, 1-Tridecanol, 1-Tetradecanol, 1-Pentadecanol, 1-Hexadecanol, 1-Heptadecanol, 1-Octadecanol, 1-Nonadecanol, 1-Eicosanol
oder
mehrwertiger Alkohole mit 2 bis 18 C-Atomen wie Ethylenglycol, 1,2-Propylenglycol, 1,3-Propylenglycol, Glycerin, Pentaerythrit, Zuckeralkoholen wie Erythritol, Sorbitol, Glucitol, Mannit, Monosaccharide wie Tetraosen, z.B. Erythrose oder . Threose, Pentaosen, z.B. Arabinose, Ribose, Lyxose, Xylose, Hexaosen wie Allose, Altrose, Galactose, Mannose, Gulose, Idose, Glucose, Talose, Fructose, Oligosaccharide wie Maltose, Raffinose, Sucrose, Maltooligosaccharide oder Lactose, wobei die weiteren OH-Gruppen der mehrwertigen Alkohole mit aliphatischen, gesättigten oder ungesättigten Carbonsäuren verestert sein können oder
von ein- oder mehrfach hydroxy-substituierten Fettsäuren, beispielsweise 8-Hydroxy-5,6-octadiensäure, die ihrerseits mit den oben genannten einwertigen aliphatischen Alkoholen oder mehrwertigen Alkoholen verestert sind.

Aliphatische, gesättigte oder ungesättigte Carbonsäuren im Sinne der Erfindung sind gesättigte oder ungesättigte lineare Carbonsäuren mit 2 bis 26 Kohlenstoffatomen, insbesondere aber Essigsäure, Propionsäure, Buttersäure, Pentansäure, Hexansäure, Heptansäure, Octansäure, Nonansäure, 2*E*-Nonensäure, Decansäure, 2E-Decensäure, 2*E*,4*E*-Decadiensäure, 2*E*,4*Z*-Decadiensäure, 2*E*,4*Z*,7*Z*-Decatriensäure, 3*Z*,5*E-*Decadiensäure, die Stereoisomere der 4,5-Dihydroxy-2-decensäure, die Stereoisomere der 4,5-Epoxy-2-decensäure, 3*Z*,5*E*,7*Z*-Decatriensäure, Deca-2,8-dien-4,6-diinsäure, Deca-2-en-4,6,8-triinsäure, Undecansäure, Dodecansäure, Tridecansäure, Tetradecansäure, Pentadecansäure, Hexadecansäure, 9*E*- oder 9*Z-*Hexadecensäure, Heptadecansäure, Octadecansäure, 9*E*- oder 9*Z*- oder 11*E*- oder 11*Z*-Octadecensäure, die verschiedenen geometrischen Isomeren der 9,12-Octadecadiensäure, der 6,9,12-Octadecatriensäure, der 9,12,15-Octadecatriensäure, der 6,9,12,15-Octadecatetraensäure, die Nonadecansäure, die Eicosansäure, die verschiedenen geometrischen Isomeren der Eicosaensäure, der 11,14-Eicosadiensäure, der 8,11,14-Eicosatriensäure, der 5,8,11,14-Eicosatetraesäure, der 5,8,11,14,17-Eicosapentaensäure, der 10,13,16-Docosatriensäure, der 7,10,13,16-Docosatetrensäure, der 4,7,10,13,16-Docosapentaensäure und der 4,7,10,13,16,19-Docosahexaensäure.

Die 2*E*,4*Z*-Decadiensäureester im Sinne der Erfindung können bevorzugt in Form natürlicher oder angereicherter prozessierter Triglyceride, beispielsweise aus fetten Ölen aus Stillingia (*Sapiuin sebiferum*), *Sebastiana ligustra* oder *Sebastiana commersoniana*), oder als Methyl- oder Ethylester vorliegen. Besonders bevorzugt ist ein durch enzymatische Umesterung von Stillingiaöl in Ethanol und anschließende Destillation erhaltene C₁₀-Frahion, **gekennzeichnet dadurch, dass** sie mindestens 80 Gew.-% Ethyl-2*E*,4*Z*-decadienoat enthält.

Überraschenderweise zeigt das erfindungsgemäße 2*E*,4*Z*-Decadiensäure-*N*-isobutylamid bei der sensorischen Untersuchung im Gegensatz zum isomeren 2*E*,4*E-*Decadiensäure-*N*-isobutylamid einen angenehmen, stark scharfen und warmen Geschmackseindruck, der an Ethanol erinnert und relativ lang anhält. Dabei sind keine weiteren sensorischen Eindrücke zu erkennen, so dass das Profil sehr neutral ist.

Das natürliche Vorkommen von trans-Pellitorin (2*E*,4*E*-Decadiensäure-*N*-isobutylamid) wurde vielfach in der Literatur beschrieben. Das Amid ist relativ weit verbreitet und kommt z.B. in Pfeffer vor (Übersicht G.M. Strunz, Stud. Nat. Prod. Chem, 2000, Band 24 (Bioactive Natural Products (Part E)), Seiten 683-738). Sein sensorischer Eindruck wurde als vorwiegend betäubend beschrieben (vgl. z.B. J. Agric. Food Chem., Jahrg. 1981, Bd. 29, Seiten 115ff. oder Fitoterapia, Jahrg. 2001, Band 72, Seiten 197ff.), wie auch in eigenen Vergleichsversuchen festgestellt werden konnte.

Dagegen wurde 2*E*,4*Z*-Decadiensäure-*N*-isobutylamid erst kürzlich in Estragon gefunden, wie in Phytochemistry, Jahrg. 2001, Bd. 58, Seiten 1083-1086 beschrieben.

Die Erfindung betrifft daher außerdem die Verwendung von 2*E*,4*Z*-Decadiensäureisobutylamid als Aromastoff, bevorzugt als Scharfstoff oder Aromastoff mit einem wärmeerzeugenden Effekt, insbesondere bevorzugt als Scharfstoff oder Aromastoff mit einem wärmeerzeugenden Effekt in der Ernährung, der Mundhygiene oder dem Genuss dienenden Zubereitungen.

Ein weiterer Gegenstand der vorliegenden Erfindung sind Zubereitungen, Halbfertigwaren und Riech-, Aroma- und Geschmackstoffkompositionen, enthaltend 2*E*,4*Z*-Decadiensäure-*N*-isobutylamid.

Selbstverständlich kann 2*E*,4*Z*-Decadiensäure-*N*-isobutylamid auch in kosmetischen oder dermatologischen Zubereitungen zur Wärmeerzeugung auf der Haut verwendet werden.

In einer besonders bevorzugten Ausführung der Erfindung wird 2*E*,4*Z*-Decadiensäure-*N*-isobutylamid in Kombination mit anderen scharf schmeckenden und/oder wärmeerzeugende Substanzen oder auch scharf schmeckenden pflanzlichen Extrakten verwendet. Auf diese Weise kann ein besonders abgerundetes sensorisches Profil erreicht werden. Insbesondere die Kombination des 2*E*,4*Z*-Decadiensäure-*N*-isobutylamids mit einem scharf schmeckenden pflanzlichen Extrakt im Verhältnis 0,01 zu 1 bis 100 zu 1, bevorzugt 0,1 zu 1 bis 10 zu 1 erzeugt ein angenehmes sensorisches Profil.

Andere scharf schmeckende und/oder wärmeerzeugende Substanzen können z.B. sein Capsaicin, Dihydrocapsaicin, Gingerol, Paradol, Shogaol, Piperin, Carbonsäure-N-vanillylamide, insbesondere Nonansäure-N-vanillylamid, 2-Alkensäureamide, insbesondere 2-Nonensäure-N-isobutylamid, Pellitorin oder Spilanthol, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 4-Acyloxy-3-methoxybenzylalkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimeihoxybenzylalkohol, Alkylether von 3-Ethoxy-4-hydroxybenzylalkohol, Alkylether von 3,4-Methylendioxybenzylall:ohol, (4-Hydroxy-3-methoxyphenyl)essigsäureamide, insbesonders (4-Hydroxy-3-methoxyphenyl)essigsäure-N-n-octylamid, Ferulasäure-phenethylamiden, Nicotinaldehyd, Methylnicotinat, Propylnicotinat, 2-Butoxyethylnicotinat, Benzylnicotinat, 1-Acetoxychavicol, Polygodial oder Isodrimeninol.

Scharf schmeckende pflanzliche Extrakte können alle für die Ernährung geeigneten pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper- ssp.,* insbesondere *Piper nigrum*), Wasserpfefferextrakt (*Polygonum ssp.,* insbesondere *Polygonum hydropiper*), Extrakte aus *Allium ssp.* (insbesondere Zwiebel und Knoblauchextrakte), Extrakte aus Rettich (*Raphanus ssp.*), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Ancyclus ssp.,* insbesondere *Anacylcus pyrethrum* L.), Sonnenhutextrakte (*Echinaceae ssp.*), Extrakten aus Szechuan-Pfeffer (*Zanthoxylum ssp.,* insbeosndere *Zanthoxylum piperitum*), Spilanthesextrakt (*Spilanthes ssp.,* insbesondere *Spilanthes acmella*), Chiliextrakt (*Capsicum ssp.,* insbesondere *Capsicum frutescens*), Paradieskömer-Extrakt (*Aframomum ssp.,* insbesondere *Aframomum melegueta* [Rose] K. Schum.), Ingwerextrakt (*Zingiber ssp.,* insbesondere *Zingiber officinale*) und Galangaextrakt (*Kaempferia galanga* oder *Alpinia galanga*).

Die scharf schmeckenden pflanzlichen Extrakte können aus den entsprechenden frischen oder getrockneten Pflanzen oder Pflanzenteilen, insbesondere aber aus weißen, grünen oder schwarzen Pfefferkörnern, Wasserpfefferkörnern, Zwiebeln und Knoblauch, Rettichwurzel, Meerrettich, Senfkömem, Sonnenhutwurzeln, Bertramwurzel, Pflanzenteilen der *Zatzthoxylum*-Arten, Pflanzenteilen der Spilanthes-Arten, Chilischoten, Paradieskörnern oder Ingwer- oder Galangawurzeln gewonnen werden, dergestalt, dass man die getrockneten Pflanzenteile, die vorzugsweise vorher zerkleinert wurden, mit einem für Nahrungs- und Genussmittel geeigneten Lösungsmittel, vorzugsweise aber Ethanol, Wasser, Hexan oder Heptan oder Ethanol/Wasser-Gemischen, bei 0°C bis zum Siedepunkt des jeweiligen Lösungsmittels oder Gemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise mit Wasserdampf bei Drücken von 0,01 mbar bis Normaldruck behandelt und/oder in einem für Nahrungs- und Genussmittel geeigneten Lösungsmittel aufgenommen werden.

Ein für Nahrungs- und Genussmittel geeignetes Lösungsmittel kann beispielsweise sein: Wasser, Ethanol, Methanol, Propylenglycol, Glycerin, Aceton, Dichlormethan, Diethylether, Hexan, Heptan, Triacetin, pflanzliche Öle oder Fette, oder superkritisches Kohlendioxid oder Gemische der vorgenannten Lösungsmittel.

Weiterer Gegenstand der Erfindung sind der Ernährung oder dem Genuss dienende Zubereitungen, enthaltend 2*E*,4*Z*-Decadiensäure-*N*-isobutylamid in einer wirksamen Menge und gegebenenfalls andere übliche Grund-, Hilfs- und Zusatzstoffe für Nahrungs- und Genussmittel. Sie enthalten in der Regel 0,000001 Gew.-% bis 10 Gew.-%, bevorzugt 0,0001 bis 1 Gew.-%, insbesondere aber 0,0001 Gew.-% bis 0,1 Gew.-%, bezogen auf das Gesamtgewicht der Zubereitung, an 2*E*,4*Z*-Decadiensäure-*N*-isobutylamid. Weitere übliche Grund-, Hilfs- und Zusatzstoffe für Nahrungs- oder Genussmittel können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die der Ernährung oder dem Genuss dienenden Zubereitungen im Sinne der Erfindung sind z.B. Backwaren (z.B. Brot, Trockenkekse, Kuchen, sonstiges Gebäck), Süßwaren (z.B. Schokoladen, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, Fleischprodukte (z.B. Schinken, Frischwurst- oder Rohwurstzubereitungen), Eier oder Eiprodukte (Trockenei, Eiweiß, Eigelb), Getreideprodukte (z.B. Frühstückscerealien, Müsliriegel), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen), Gemüsezubereitungen (z.B. Ketchup, Soßen, Trockengemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), Fertiggerichte und Suppen, Gewürze, Würzmischungen sowie insbesondere Aufstreuwürzungen (Seasonings), die im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Besonders vorteilhaft hat sich auch erwiesen, dass 2*E*,4*Z*-Decadiensäure-*N-*isobutylamid, insbesondere die erfindungsgemäße Kombination von 2*E*,4*Z-*Decadiensäure-*N*-isobutylamid mit scharf schmeckenden pflanzlichen Extrakten, den scharfen Geschmack von Alkohol in alkoholischen Getränken oder Zubereitungen aus alkoholischen Getränken imitieren können und es damit möglich ist, den Alkoholgehalt in alkoholischen Getränken oder in Zubereitungen aus alkoholischen Getränken bei gleichbleibender sensorischer Beurteilung niedriger einzustellen.

Besonders vorteilhaft hat sich auch erwiesen, dass 2*E*,4*Z*-Decadiensäure-*N-*isobutylamid den scharfen Geschmack von Capsaicin, Dihydrocapsaicin und Nonivamid imitieren können und es damit möglich ist, den Capsaicingehalt in den der Ernährung oder dem Genuss dienenden Zubereitungen bei gleichbleibender sensorischer Beurteilung niedriger einzustellen.

Eine weitere bevorzugte Ausführungsform der Erfindung sind der Mundhygiene dienende Zubereitungen, insbesondere Zahnpflegemittel wie Zahnpasten, Zahngele, Zahnpulver, Mundwässer, Kaugummis und andere Mundpflegemittel, enthaltend 2*E*,4*Z*-Decadiensäure-*N*-isobutylamid in einer wirksamen Menge und gegebenenfalls andere übliche Grund-, Hilfs- und Zusatzstoffe für solche Zubereitungen. Sie enthalten in der Regel 0,000001 Gew.-% bis 10 Gew.-%, bevorzugt 0,0001 bis 1 Gew.- %, insbesondere aber 0,0001 Gew.-% bis 0,1 Gew.-%, bezogen auf das Gesamtgewicht der Zubereitung, an 2*E*,4*Z*-Decadiensäure-*N*-isobutylamid. Weitere übliche Grund-, Hilfs- und Zusatzstoffe für die der Mundhygiene dienenden Zubereitungen können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Zahnpflegemittel, die 2*E*,4*Z*-Decadiensäure-*N*-isobutylamid enthalten, bestehen im allgemeinen aus einem abrasiven System (Schleif- oder Poliermittel), wie z.B. Kieselsäuren, Calciumcarbonaten, Calciumphosphaten, Alumiuniumoxiden und/oder Hydroxylapatiten, aus oberflächenaktiven Substanzen, wie z.B. Natriumlaurylsulfat, Natriumlaurylsarcosinat und/oder Cocamidopropylbetain, aus Feuchthaltemitteln, wie z.B. Glycerin und/oder Sorbit, aus Verdickungsmitteln, wie z.B. Carboxymethylcellulose, Polyethylenglycolen, Carrageenanen und/oder Laponiten^{®}, aus Süßstoffen, wie z.B. Saccharin, aus Stabilisatoren und aus aktiven Wirkstoffen, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat.

Kaugummis, enthaltend 2*E*,4*Z*-Decadiensäure-*N*-isobutylamid, bestehen im Allgemeinen aus einer Kaugummibase, d.h. einer beim Kauen plastische werdenden Kaumasse, aus Zuckern verschiedener Arten, Zuckeraustauschstoffen, Süßstoffen, Zuckeralkoholen, Feuchthaltemitteln, Verdickern, Emulgatoren, Aromen und Stabilisatoren.

Die erfindungsgemäßen Zubereitungen, enthaltend 2*E*,4*Z*-Decadiensäure-*N*-isobutylamid, können dergestalt hergestellt werden, dass 2*E*,4*Z*-Decadiensäure-*N*-isobutylamid als Substanz, als Lösung oder in Form eines Gemisches mit einem festen oder flüssigen Trägerstoff in die der Ernährung, der Mundhygiene oder dem Genuss dienenden Zubereitungen eingearbeitet werden. Vorteilhafterweise können die als Lösungen vorliegenden erfindungsgemäßen Zubereitungen, enthaltend 2*E*,4*Z-*Decadiensäure-*N*-isobutylamid, auch durch Sprühtrocknung in eine feste Zubereitung überführt werden.

Zur Herstellung der Zubereitungen können in einer weiteren bevorzugten Ausführungsform die 2*E*,4*Z*-Decadiensäure-*N*-isobutylamid und gegebenenfalls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere Ausführungsform besteht darin, dass 2E,4Z-Decadiensäureisobutylamid vorher mit geeigneten Komplexbildnern, beispielsweise mit Cyclodextrinen oder Cyclodextrinderivaten, bevorzugt β-Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Ei, Knochen, Knorpel, Fisch, Krusten- und Schalentiere, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit, Mannitol, Xylitol), natürliche oder gehärtete Fette (z.B. Talg, Schmalz, Palmfett, Kokosfett, gehärtetes Pflanzenfett), fette Öle (z.B. Sonnenblumenöl, Erdnussöl, Maiskeimöl, Distelöl, Olivenöl, Walnussöl, Fischöl, Sojaöl, Sesamöl), Fettsäuren oder deren Salze (z.B. Kaliumstearat, Kaliumpalmitat), proteinogene oder nicht-proteinogene Aminosäuren und verwandte Verbindungen (z.B. Taurin, Kreatin, Kreatinin), Peptide, native oder prozessierte Proteine (z.B. Gelatine), Enzyme (z.B. Peptidasen, Glucosidasen, Lipasen), Nukleinsäuren, Nucleotide (Inositolphosphat), geschmacksmodulierende Stoffe (z.B. Natriumglutamat, 2-Phenoxypropionsäure), Emulgatoren (z.B. Lecithine, Diacylglycerole), Stabilisatoren (z.B. Carageenan, Alginat, Johannisbrotkernmehl, Guarkernmehl), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin), Süßstoffe (z.B. Saccharin, Cyclamat, Aspartam, Neotam, Neohesperidindi-hydrochalkon), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, sowie Riechstoffe, synthetische, natürliche oder naturidentische Aroma- und Geschmackstoffe.

Bevorzugt können die erfindungsgemäßen Zubereitungen auch noch eine Aromakomposition enthalten, um den Geschmack und/oder Geruch der Zubereitung abzurunden und zu verfeinern. Geeignete Aromakompositionen enthalten z.B. synthetische, natürliche oder naturidentische Aromastoffe sowie Riechstoffe, insbesondere aber auch andere scharf schmeckende und/oder wärmeerzeugende Substanzen oder Pflanzenextrakte.

Ein weiterer Gegenstand der Erfindung ist die Verwendung der erfindungsgemäßen Zubereitungen als Halbfertigwaren zur Aromatisierung von daraus gefertigten Zubereitungen als Fertigwaren.

### Beispiele

### Beispiel 1 Darstellung von 2E,4Z-Decadiensäure-N-isobutylamid (cis-Pellitorin) durch enzymatische Umsetzung mit Ethyl-2E,4Z-decadienoat

10 g Ethyl-2*E*,4*Z*-decadienoat, 4,7 g Chirazym L-2 (c.-f., C2, lyo., Katalog-Nr. 1859242, Roche Diagnostics, Basel, Schweiz), 4 g Isobutylamin wurden bei 55°C 4 Tage gerührt. Der Ansatz wurde mit 100 ml Diethylether versetzt und filtriert; das Filtrat wurde im Vakuum eingedampft (Rohausbeute 15,2 g). Das Produkt wurde in 10 %iger KOH/Methanol (1:1-Gemisch) 45 min bei Raumtemperatur gerührt, mit Ether extrahiert, die etherische Phase über Natriumsulfat getrocknet, filtriert und das Filtrat eingedampft. Das Rohprodukt wurde an Kieselgel 60 chromatografiert (Eluent Hexan/Ethylacetat 10:1). Ausbeute 9,1 g (GC: 99,4 %); ¹H-NMR (CDCl₃; 200 MHz): 7,56 (1H, ddd, 11,5 Hz, 14,9 Hz, 1.0 Hz), 6,08 (1H, dddd 11,5 Hz, 10,8 Hz, 1,4 Hz, 0,6 Hz), 5,82 (1H, d, 11,5 Hz), 5,79 (1H, dtd 10,8 Hz, 7,8 Hz, 0,9 Hz), 5,50 (1H, bs), 3,18 (2H, dd, 6,8 Hz, 6,1 Hz), 2,36-2,22 (2H, m), 1,81 (1H, m, 6,7 Hz), 1,50 - 1,22 (6H,m), 0,93 (6H, d, 6,7 Hz), 0,88 (3H, m) ppm; ¹³C-NNIR (CDCl₃; 50 MHz): 166,34 (C), 140,07 (CH), 135,76 (CH), 126,28 (CH), 123,78 (CH), 46,96 (CH₂), 31,41 (CH₂), 29,14 (CH₂), 28,63 (CH), 28,15 (CH₂), 22,52 (CH₂), 20,15 (CH₃), 14,02 (CH₃) ppm.

### Beispiel 2 Darstellung von technischem 2E,4Z-Decadiensäure-N-isobutylamid (cis-Pellitorin) durch enzymatische Umsetzung mit Ethyl-2E,4Z-decadienoat in Toluol

100 g Ethyl-2*E*,4*Z*-decadienoat und 40 g Chirazym L-2 (c.-f., C2, lyo., Katalog-Nr. 1859242, Roche Diagnostics, Basel, Schweiz) wurden in 50 ml Toluol vorgelegt und unter Rühren 40 g Isobutylamin bei 55°C portionsweise über 4 Tage hinzugefügt. Nach Filtration und schonendem Abdestillieren des Toluols im Vakuum erhält man ein Produkt mit 81 % cis-Pellitorin (GC).

### Beispiel 3 Darstellung eines Präparates, enthaltend 2E,4Z-Decadiensäure-N-isobutylamid (cis-Pellitorin) durch enzymatische Umsetzung mit Stillingiaöl

100 g Stillingiaöl, 20 g Chirazym L-2 (c.-f., C2, lyo., Katalog-Nr. 1859242, Roche Diagnostics, Basel, Schweiz) und 40 g Isobutylamin wurden bei 45°C 72 h gerührt und das Enzym abfiltriert. Das Rohprodukt (116 g) enthält 2,5 % (GC) cis-Pellitorin. Daraus konnte durch Molekulardestillation (0,12 mbar, 150°C) ca. 50 g einer Fraktion mit 6,2 % cis-Pellitorin und durch erneute Molekulardestillation dieser Fraktion eine neue ca. 12 g-Fraktion mit 17,5 % cis-Pellitorin gewonnen werden.

### Beispiel 4 Darstellung von 2E,4E-Decadiensäure-N-isobutylamid (trans-Pellitorin) (Vergleich)

277 mg 2*E*,4*Z*-Decadiensäure-*N*-isobutylamid aus Beispiel 1 wurden mit 29 mg Iod in 10 ml Toluol eine Stunde bei Raumtemperatur gerührt. Die Mischung wurde an Kieselgel 60 mit dem Eluenten Hexan/Ethylacetat 5:1 chromatografiert. Ausbeute: 61 mg (> 95 %, NMR); ¹H-NMR (CDCl₃; 200 MHz): 7,19 (1H, dd, 14,9 Hz, 9,7 Hz), 6,13 (1H, dd 15,1 Hz, 9,6 Hz), 6,07 (1H, dd, 15,1 Hz, 6,4 Hz), 5,75 (1H, d 14,9 Hz), 5,50 (1H, bs), 3,17 (2H, dd, 6,9 Hz, 6,1 Hz), 2,14 (2H, dd, 7Hz, 6,4 Hz), 1,80 (1H, m, 6,7 Hz), 1,42 (2H, m, 7,1 Hz), 1,37 - 1,22 (4H, m), 0,93 (6H, d, 6,7 Hz), 0,89 (3H, m) ppm.

### Beispiel 5 Verkostung von cis-Pellitorin

Die zu verkostende Substanz wird in Ethanol gelöst und die ethanolische Lösung dann mit 11 %iger Zuckerlösung verdünnt (Endkonzentration: c). Zur Verkostung werden jeweils ca. 5 ml der Zuckerlösung heruntergeschluckt. Wenn der Schwellenwert der Substanz bekannt ist, wird für die Verkostung ein Wert knapp über dem Schwellenwert gewählt. Eine Gruppe von 6 - 8 Prüfern hat die Lösungen verkostet.
a) Profil 2*E*,4*Z*-Decadiensäure-*N*-isobutylamid (cis-Pellitorin):
   c = 10 ppm: deutliches Wärmegefühl, direkt scharf, typische Alkoholschärfe.

### Vergleichsbeispiele

b) Profil Dihydrocapsaicin:
   c = 100 ppb: leicht verzögert einsetzende Wirkung im Rachenraum, brennend, aggressiv, keine Wärmeentwicklung.
   c) Profil 2*E*,4*E*-Decadiensäure-*N*-isobutylamid (trans-Pellitorin) c = 10 ppm: speichelfördernd, fettig, fruchtig, leicht kribbelnd, nicht scharf

### Beispiel 6 Anwendung in einem Apfelschnaps als Alkoholverstärker

- 14,90 l: Alkohol 96 Vol.-%
- 5,2 l: Aroma (Natürliches Apfel-Fruchtsaft-Likör-Aroma, 15 % Vol., enthält 0,01 Gew.-% cis-Pellitorin)
- 27 kg: Zuckersirup
- 1 kg: Citronensäure-Monohydrat
Auffüllen mit Wasser, demineralisiert auf 1001; Gesamtmenge 1001

### Beispiel 7 Anwendung in Kombination mit einem scharfen Pflanzenextrakt als Alkoholverstärker

- 14,90 l: Alkohol 96 Vol.-%
- 5,2 l: Aroma (Natürliches Apfel-Fruchtsaft-Likör-Aroma, 15 % Vol., enthält 0,0025 Gew.-% cis-Pellitorin und 0,0075 Gew.-% Paradies- kömerextrakt)
- 27 kg: Zuckersirup
- 1 kg: Citronensäure-Monohydrat
Auffüllen mit Wasser, demineralisiert auf 1001; Gesamtmenge 1001

### Beispiel 8 Anwendung in einer Zahnpasta als Aromastoff

| **Teil** | **Inhaltsstoff** | **Einsatz in Gew.-%** |
|---|---|---|
| A | demineralisiertes Wasser | 22,00 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aroma, enthaltend 0,1 % cis-Pellitorin | 1 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Beispiel 9 Anwendung in einem zuckerfreien Kaugummi als Aromastoff

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Aroma, enthaltend 0,1 % cis-Pellitorin | 1 |

Teile A bis D werden gemischt und intensiv geknetet. Die Rohmasse kann z.B. in Form von dünnen Streifen zu verzehrsfertigen Kaugummis verarbeitet werden.

### Beispiel 10 Anwendung in einem Mundwasser als Aromastoff

| **Teil** | **Inhaltsstoff** | **Gehalt (%)** |
|---|---|---|
| A | Ethanol | 10,00 |
| | Cremophor^{®} CO 40 (BASF, Detergenz) | 1,00 |
| | Benzoesäure | 0,12 |
| | Aroma, enthaltend 0,4 % cis-Pellitorin | 0,25 |
| B | demineralisiertes Wasser | 83,46 |
| | Sorbitol, 70% | 5,00 |
| | Natriumsaccharin 450 | 0,07 |
| | L-Blue 5000 e.c., 1 % in Wasser (Farbstoff) | 0,10 |

Die Inhaltsstoffe der Teile A und B werden jeweils für sich gemischt. Teil B wird langsam in Teil A eingerührt, bis die Mischung homogen ist.

## Patentansprüche

1. Verfahren zur Herstellung von 2*E*,4*Z*-Decadiensäure-*N*-isobutylamid (cis-Pellitorin), **dadurch gekennzeichnet, dass** man einen 2*E*,4*Z*-Decadiensäureester mit Isobutylamin in Gegenwart eines Enzyms mit Lipaseaktivität umsetzt, das Gemisch mit nicht umgesetztem 2*E*,4*Z*-Decadiensäureester gegebenenfalls einer Verseifung unterzieht und anschließend das Gemisch mit physikalisch chemischen Methoden aufreinigt.

2. Mischung enthaltend mindestens 80 Gew.% 2*E*,4*Z*-Decadiensäure-*N*-isobutylamid und mindestens zwei weitere *N*-Isobutylamiden der Decansäure, 2*E*-Decensäure, 2*E*,4*E*-Decadiensäure, 2*Z*,4*E*-Decadiensäure, 2*Z*,4*Z-*Decadiensäure, 2*E*,4*Z*,7*Z*-Decatriensäure, 3*Z*,5*E*-Decadiensäure oder 3*Z*,5*E*,7*Z*-Decatriensäure.

3. Verwendung von 2*E*,4*Z*-Decadiensäure-*N*-isobutylamid oder einer Mischung nach Anspruch 2 als Aromastoff.

4. Verwendung nach den Anspruch 3, wobei Aromastoff Scharfstoff oder Aromastoff mit einem wärmeerzeugenden Effekt bedeutet.

5. Verwendung nach Anspruch 3 oder 4 in der Ernährung oder dem Genuss dienenden Zubereitungen.

6. Verwendung nach Anspruch 3 oder 4 in der Mundhygiene dienenden Zubereitungen.

7. Der Ernährung, der Mundhygiene oder dem Genuss dienende Zubereitungen, enthaltend 2*E*,4*Z*-Decadiensäureisobutylamid in einer wirksamen Menge und mindestens eine weitere scharf schmeckende oder wärmeerzeugende Substanz oder eine Mischung nach Anspruch 2.

8. Zubereitungen nach Anspruch 7, wobei die weitere scharf schmeckende oder wärmeerzeugende Substanz ausgewählt ist aus der Gruppe bestehend aus Capsaicin, Dihydrocapsaicin, Gingerol, Paradol, Shogaol, Piperin, Carbonsäure-N-vanillylamide, insbesondere Nonansäure-N-vanillylamid, 2-Alkensäureamide, insbesondere 2-Nonensäure-N-isobutylamid, Pellitorin oder Spilanthol, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 4-Acyloxy-3-methoxybenzylalkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesonders (4-Hydroxy-3-methoxyphenyl)essigsäure-N-n-octylamid, Ferulasäure-phenethylamiden, Nicotinaldehyd, Methylnicotinat, Propylnicotinat, 2-Butoxyethylnicotinat, Benzylnicotinat, 1-Acetoxychavicol, Polygodial oder Isodrimeninol.

9. Zubereitungen nach Anspruch 7, enthaltend mindestens einen scharf schmeckenden pflanzlichen Extrakt.

10. Zubereitungen nach Anspruch 7, enthaltend mindestens eine weitere scharf schmeckende oder wärmeerzeugende Substanz und mindestens einen scharf schmeckenden pflanzlichen Extrakt.

11. Als Halbfertigwaren vorliegende Zubereitungen nach mindestens einem der Ansprüche 7 bis 10.

12. Als Riech-, Aroma- und Geschmacksstoffkompositionen sowie Würzmischungen vorliegende Zubereitungen nach mindestens einem der Ansprüche 7 bis 11.

## Claims

1. Process for the production of 2*E*,4*Z*-decadienoic acid-*N*-isobutylamide (cis-Pellitorin), **characterised in that** a 2*E*,4*Z*-decadienoic acid ester is converted with isobutylamine in the presence of an enzyme with lipase activity, the mixture undergoes a saponification, as appropriate, with non-converted 2*E*,4*Z*-decadienoic acid ester, and the mixture is then purified with physico-chemical methods.

2. Mixture containing at least 80% by weight of 2*E*,4*Z*-decadienoic acid-N-isobutylamide and at least two further *N*-isobutylamides of decenoic acid, 2*E*-decenoic acid, 2*E*,4*E*-decadienoic acid, 2*Z*,4*E*-decadienoic acid, 2*Z*,4*Z-*decadienoic acid, 2*E*,4*Z*,7*Z*-decatrienoic acid, 3*Z*,5*E*-decadienoic acid or 3*Z*,5*E*,7*Z*-decatrienoic acid.

3. Use of 2*E*,4*Z*-decadienoic acid-*N*-isobutylamide or a mixture according to Claim 2 as an aromatic substance.

4. Use according to Claim 3, wherein "aromatic substance" signifies a pungent substance or aromatic substance with a heat-producing effect.

5. Use according to Claim 3 or 4 in preparations serving as foodstuffs or luxury foodstuffs.

6. Use according to Claim 3 or 4 in preparations serving the purpose of oral hygiene.

7. Preparations serving as foodstuffs, serving the purpose of oral hygiene, or serving as luxury foodstuffs, containing 2*E*,4*Z*-decadienoic acid isobutylamide in an effective quantity and at least one further pungent-tasting or heat-producing substance or a mixture according to Claim 2.

8. Preparations according to Claim 7, wherein the further pungent-tasting or heat-producing substance is selected from the group consisting of capsaicin, dihydrocapsaicin, gingerol, paradol, shogaol, piperin, carboxylic acid-*N*-vanillylamides, in particular nonanoic acid-*N*-vanillylamide, 2-alkenoic acid amides, in particular 2-nonenoic acid-*N*-isobutylamide, pellitorin or spilanthol, 2-nonenoic acid-*N*-4-hydroxy-3-methoxyohenylamide, alkyl ethers of 4-hydroxy-3-methoxybenzyl alcohol, in particular 4-hydroxy-3-methoxybenzyl-n-butyl ether, alkyl ethers of 4-acyloxy-3-methoxybenzyl alcohol, in particular 4-acetyloxy-3-methoxybenzyl-n-butyl ether and 4-acetyloxy-3-methoxybenzyl-n-hexylether, alkyl ethers of 3-hydroxy-4-methoxybenzyl alcohol, alkyl ethers of 3,4-dimethoxy benzyl alcohol, alkyl ethers of 3-ethoxy-4-hydroxybenzyl alcohol, alkyl ethers of 3,4-methylene dioxybenzyl alcohol, (4-hydroxy-3-methoxyphenyl)acetic acid amides, in particular (4-hydroxy-3-methoxyphenyl)acetic acid-N-n-octyl amide, ferulic acid-phenethylamides, nicotine aldehyde, methyl nicotinate, propyl nicotinate, 2-butoxyethyl nicotinate, benzyl nicotinate, 1-acetoxychavicol, polygodial, or isodrimeninol,

9. Preparations according to Claim 7, containing at least one sharp-tasting plant extract.

10. Preparations according to Claim 7, containing at least one further sharp-tasting or heat-producing substance and at least one sharp-tasting plant extract.

11. Preparations, present as semi-finished products, according to at least one of Claims 7 to 10.

12. Preparations, present as scent compositions, aromatic substance compositions and flavouring substance compositions and spice mixtures, according to at least one of Claims 7 to 11.

## Revendications

1. Procédé de fabrication de N-isobutylamide d'acide 2E,4Z-décadiénique (cis-pellitorine), **caractérisé par le fait que** l'on fait réagir un ester d'acide 2E,4Z-décadiénique avec de l'isobutylamine en présence d'une enzyme à activité lipasique, le mélange contenant l'ester d'acide 2E,4Z-décadiénique non transformé est soumis, le cas échéant, à une saponification et le mélange est ensuite purifié par des méthodes physico-chimiques.

2. Mélange contenant au moins 80% en poids de N-isobutylamide d'acide 2E,4Z-décadiénique et au moins deux autres N-isobutylamides de l'acide décaztoïque, l'acide 2E-décénique, l'acide 2E,4E-décadiénique, l'acide 2Z,4E-décadiénique, l'acide 2Z,4Z-décadiénique, l'acide 2E,4Z,7Z-décatriène, l'acide 3Z,5E-décadiénique ou l'acide 3Z,5E,7Z-décatriène.

3. Utilisation de N-isobutylamide d'acide 2E,4Z-décadiénique ou d'un mélange selon la revendication 2 en tant que substance aromatique.

4. Utilisation selon la revendication 3, "substance aromatique" signifiant "substance piquante" ou "substance aromatique ayant un effet de production de chaleur".

5. Utilisation selon la revendication 3 ou 4 dans des préparations destinées à l'alimentation ou à la consommation.

6. Utilisation selon la revendication 3 ou 4 dans des préparations destinées à l'hygiène buccale.

7. Préparations destinées à l'alimentation, à l'hygiène buccale ou à la consommation, contenant de l'isobutylamide d'acide 2E,4Z-décadiénique en quantité active et au moins une autre substance à goût piquant ou produisant de la chaleur ou un mélange selon la revendication 2.

8. Préparations selon la revendication 7, l'autre substance à goût piquant ou produisant de la chaleur étant choisie dans le groupe comprenant la capsaïcine, la dihydrocapsaïcine, le gingérol, le paradol, le shogaol, la pipérine, le N-vanillylamide d'acide carboxylique, notamment le N-vanillylamide d'acide nonanoïque, des amides d'acide 2-alcénoïque, notamment le N-isobutylamide d'acide 2-nonanoïque, la pellitorine ou le spilanthol, le N-4-hydroxy-3-méthoxyphénylamide d'acide 2-nonanoïque, des alkyléthers d'alcool 4-hydroxy-3-méthoxybenzyl, notamment le 4-hydroxy-3-méthoxybenzyl-n-butyléther, des alkyléthers d'alcool 4-acyloxy-3-méthoxybenzyl, notamment le 4-acétyloxy-3-méthoxybenzyl-n-butyléther et le 4-acétyloxy-3-méthoxybenzyl-n-hexyléther, des alkyléthers d'alcool 3-hydroxy-4-méthoxybenzyl, des alkyléthers d'alcool 3,4-diméthoxybenzyl, des alkyléthers d'alcool 3-éthoxy-4-hydroxybenzyl, des alkyléthers d'alcool 3,4-méthylene-dioxybenzyl, des amides d'acide (4-hydroxy-3-méthoxyphényl)acétique, notamment le N-n-octylamide d'acide (4-hydroxy-3-méthoxyphényl)acétique, des phénétylamides d'acide férulique, le nicotinaldéhyde, la nicotinate de méthyle, la nicotinate de propyle, la nicotinate de 2-butoxyéthyle, la nicotinate de benzyle, le 1-acétoxychavicol, le polygodial ou l'isodriméninol.

9. Préparations selon la revendication 7, contenant au moins un extrait végétal à goût piquant.

10. Préparations selon la revendication 7, contenant au moins une autre substance à goût piquant ou produisant de la chaleur et au moins un extrait végétal à goût piquant.

11. Préparations se présentant comme des produits semi-finis selon au moins une des revendications 7 à 10.

12. Préparations se présentant comme des compositions de substances odoriférantes, aromatiques et gustatives ainsi que comme des mélanges d'épices selon au moins une des revendications 7 à 11.
